# EUROPEAN PATENT SPECIFICATION

(11) **EP 2 571 500 B1**
(45) Date of publication and mention of the grant of the patent: **27.09.2017**
(21) Application number: 11746336.4
(22) Date of filing: 17.05.2011
(51) Int. Cl.: A61K 31/34, A61P 11/00, A61P 43/00

(54) **TREATMENT FOR ALTITUDINAL HYPOXIA**
BEHANDLUNG VON HÖHENHYPOXIE
TRAITEMENT DE L'HYPOXIE ALTITUDINALE

(30) Priority: 18.05.2010 US 345748 P
(43) Date of publication of application: 27.03.2013
(73) Proprietor: Aesrx LLC, Newton, MA 02459 (US)
(72) Inventor: SWIFT, Robert, Alan, Fort Collins, CO 80528 (US)
(74) Representative: Adam, Holger
(86) International application number: PCT/US2011/036792
(87) International publication number: WO 2011/146471

(56) References cited:
- US-A1- 2002 065 232
- US-A1- 2004 157 801
- BURKI N K ET AL: "THE EFFECTS OF ACETAZOLAMIDE ON THE VENTILATORY RESPONSE TO HIGH ALTITUDE HYPOXIA", CHEST, vol. 101, no. 3, 1992, pages 736-741, XP002661130, ISSN: 0012-3692
- LI M -M ET AL R ET AL: "The protective role of 5-HMF against hypoxic injury", CELL STRESS AND CHAPERONES 2011 CELL STRESS AND CHAPERONES USA LNKD- DOI:10.1007/S12192-010-0238-2, vol. 16, no. 3, May 2011 (2011-05), pages 267-273, XP002661131, ISSN: 1355-8145
- ABDULMALIK OSHEIZA ET AL: "5-hydroxymethyl-2-furfural modifies intracellular sickle haemoglobin and inhibits sickling of red blood cells", BRITISH JOURNAL OF HAEMATOLOGY, vol. 128, no. 4, February 2005 (2005-02), pages 552-561, XP002661132, ISSN: 0007-1048 cited in the application
- WEIL W.M. ET AL.: "High-Altitude Illness and Muscle Physiology", BULLETIN OF THE NYU HOSPITAL FOR JOINT DISEASES, vol. 65, no. 1, 2007, pages 72-77,

## Description

### BACKGROUND OF THE INVENTION

### Field of the invention

The invention relates to alleviation of symptoms of hypoxia resulting from reduced oxygen levels at high altitudes.

### Summary of the related art

Hemoglobin is the oxygen transporter and regulator in blood. Each hemoglobin molecule can bind up to four oxygen atoms. The oxygen tension (in millimeters of mercury) at which the oxygen binding sites of hemoglobin are 50 percent saturated is called P₅₀. P₅₀ is inversely related to the binding affinity of hemoglobin for oxygen. Standard P₅₀ is the P₅₀ under standard conditions of 37°C, pH 7.4, carbon dioxide tension of 40mm Hg at sea level, normally 26.3 mm Hg for a resting adult. However, important physiologic effects are determined by in vivo P₅₀, which changes rapidly in responses to body temperature, carbon dioxide tension and pH.

As body temperature increases, the affinity of hemoglobin for oxygen decreases, raising the P₅₀ and facilitating oxygen release. Lactic acidosis due to muscular activity further increases this effect. This is beneficial during prolonged heavy exercise. High levels of oxygen reduce P₅₀ and increase the affinity of hemoglobin for oxygen. In contrast, low levels of oxygen increase P₅₀ and decrease the affinity of hemoglobin for oxygen.

Taken together, these factors lead to a seriously decreased affinity of hemoglobin for oxygen under conditions of exercise at oxygen-depleted high altitudes. For example, at an altitude of 3,100 m a P₅₀ of 29 mm Hg at rest and 38 mm Hg during heavy exercise have been observed. Thus, the primary limitation on oxygen transport at high altitude is impaired loading of oxygen onto hemoglobin caused by alveolar hypoxia.

Some animals indigenous to high altitudes, such as yaks, llamas and alpacas, and high altitude rodents and birds have high affinity hemoglobins having P₅₀ about 10 mm Hg lower than in related lowland species due to amino acid substitutions in the globin chain. In fact, humans with the hemoglobin mutation Andrew-Minneapolis, resulting in a P₅₀ of 17 mm Hg, have better physiologic function at high altitudes than normal subjects whose P50 is 26.3 mm Hg. However, as a result of having evolved at lower altitudes, most humans have an inappropriate increase of P₅₀ at high altitudes, resulting in debilitating symptoms of hypoxia. There is no FDA approved drug that improves (lowers) the P50 and thus increases Hb affinity for oxygen in normal subjects. However, 5-HMF was evaluated for its effects on P50 in preclinical studies using sickle cell disease (SCD) red cells. These RBCs have a diminished oxygen carrying capacity due to amino acid changes in Hb structure as a result of a genetic mutation. Abdulmalik, Br. J. Haematol. 128:552-561 (2004), teaches that 5-HMF provides in vivo protection against the lethal effects of hypoxia in a sickle cell disease mouse model, and that this is the result of a lower P50 (left shift) in the SCD Hb, thus reducing the formation of sickled red blood cells in conditions of low oxygen in the inspired air. Thus, according to Abdumalik, the beneficial effect of 5-HMF in prolonging survival in the hypoxic state is due to the inhibition of RBC sickling, a phenomenon that is unique to SCD and would not be found in normal subjects. There is a need for new treatments for alleviating the symptoms of hypoxia in normal humans at high altitudes.

N.K. Burki et al. in Chest. 1992; 101 (3):736-741 discuss the effects of acetazolamide on the ventilatory response to high altitude hypoxia.

US 2002/0065232 A1 teaches that persons encountering high altitude without being acclimatized can prevent or alleviate the symptoms of acute mountain sickness by administration of ribose one to four times a days, beginning immediately on encountering high altitudes and continuing for at least five days.

Ming-Ming Li et al. discuss in Cell Stress Chaperones 2001 May; 16(3): 267-273 the protective role of 5-HMF against hypoxic injury.

US 2004/0157801 A1 discloses anti-sickling agents. In particular, this document discloses 5-membered heterocyclic anti-sickling agents that are highly effective and non-toxic, and methods for their use. The compounds include analogues and derivatives of naturally occurring 5-hydroxymethyl-2-furfuraldehyde, 5-Ethyl-2-furfuraldehyde, 5-Methyl-2-furfuraldehyde, and 2-furfuraldehyde.

Wayne M. Weil, et al. describe in Bulletin of the NYU Hospital for Joint Diseases 2007;65(1):72-7, ,,High-Altitude Illness and Muscle Physiology" several treatments for high-altitude illness.

### BRIEF SUMMARY OF THE INVENTION

The invention provides a new treatment of altitudinal hypoxia. The invention comprises administering to a human subject encountering or about to encounter conditions of low oxygen a compound that decreases P₅₀ of hemoglobin for oxygen. The compound is administered at a dosage sufficient to provide in the subject a P₅₀ of hemoglobin from about 15 mm Hg to about 20 mm Hg under standard conditions of 37°C, pH 7.4, carbon dioxide tension of 40mm Hg at sea level. The compound is 5-hydroxymethyl-2-furfural (5-HMF).

Accordingly, the present invention relates to hydroxymethyl-2-furfural (5-HMF) for use in treating altitudinal hypoxia in a human subject, in a dosage sufficient to provide in the human subject a P₅₀ of hemoglobin from 15 mm Hg to 20 mm Hg under standard conditions.

### DETAILED DESCRIPTION OF THE PREFERRED EMBODIMENTS

The invention provides a new treatment of altitudinal hypoxia. The invention comprises administering to a human subject encountering or about to encounter conditions of low oxygen a compound that decreases P₅₀ of hemoglobin for oxygen, hence increasing the affinity of Hb for oxygen. The invention prevents, reduces or alleviates symptoms of hypoxia. The compound is administered at a dosage sufficient to provide in the subject a P₅₀ of hemoglobin from about 15 mm Hg to about 20 mm Hg under standard conditions. The compound is 5-HMF.

For purposes of the invention, the term " a human subject encountering or about to encounter conditions of low oxygen" means a person who is either under conditions of low oxygen or about to be under conditions of low oxygen. The term "low oxygen" is intended to mean a concentration of oxygen typically found at elevations at or above about 1,500 meters above sea level and especially at 3,000 or more meters above sea level. The term "standard conditions" means the conditions of an average adult at rest at about 37°C, pH 7.4, carbon dioxide tension of 40mm Hg at sea level. The term "prevents or alleviates symptoms" means to stop or reduce the development of symptoms or to eliminate or reduce existing symptoms. "Symptoms" include, without limitation, reduced oxygenation of blood, red blood cells, and/or tissues, as well as impaired mental function and/or reduced ability to exercise. Such symptoms can be measured using standard techniques, such as pulse oximetry, blood gases analysis and exercise tolerance testing.

In certain preferred embodiments, the subject is either at, or about to be transported to, an elevation of from about 1,500 meters to about 9,000 meters above sea level. In certain preferred embodiments, the subject is either at, or about to be transported to, an elevation of from about 3,000 meters to about 9,000 meters above sea level.

In certain preferred embodiments, the compound is administered at a dosage sufficient to provide in the subject a P₅₀ of hemoglobin for oxygen of from about 17 mm Hg to about 20 mm Hg under standard conditions. The dosage required to produce such a P₅₀ will depend upon several factors, such as the age, weight, health and fitness of the subject, and can be determined routinely. Preferred compounds for use in the invention are non-toxic at the concentrations to produce the desired P₅₀. For example, 5-hydroxymethyl-2-furfural is found in foods that are consumed on a daily basis, such as coffee and caramel products, at a concentration above 6 g/kg. In rats, the acute oral LD₅₀ of 5-HMF is 2.5 g/kg for males and 2.5-5.0 g/kg for females (US EPA, 1992).

In some embodiments the human is administered from 20 mg to 10,000 mg 5-HMF. In some embodiments the human is administered from 20 mg to 300 mg 5-HMF. In some embodiments the human is administered from 20 mg to 200 mg 5-HMF. In some embodiments the human is administered from 20 mg to 100 mg 5-HMF. In some embodiments the human is administered from 300 mg to 10,000 mg 5-HMF. In some embodiments the human is administered from 300 mg to 5,000 mg 5-HMF. In some embodiments the human is administered from 300 mg to 3,000 mg 5-HMF. In some embodiments the human is administered from 300 mg to 1,500 mg 5-HMF. In some embodiments the human is administered from 1,500 mg to 10,000 mg 5-HMF. In some embodiments the human is administered from 1,500 mg to 5,000 mg 5-HMF. In some embodiments the human is administered from 1,500 mg to 3,000 mg 5-HMF. In some embodiments the human is administered from 3,000 mg to 10,000 mg 5-HMF. In some embodiments the human is administered from 3,000 mg to 5,000 mg 5-HMF.

Administration of the compound can be by any suitable route, including, without limitation, parenteral, oral, sublingual, transdermal, topical, intranasal, inhalation, intratracheal, or intrarectal. In some preferred embodiments, administration is orally, by injection, or by inhalation.

The compound used in the present invention may be present in any suitable diluent, carrier or excipient.

The compound used in the present invention may form salts which are also within the scope of this invention. The term "salt(s)", as employed herein, denotes acidic and/or basic salts formed with inorganic and/or organic acids and bases. Pharmaceutically acceptable (i.e., non-toxic, exhibiting minimal or no undesired toxicological effects, physiologically acceptable) salts are preferred.

As used herein, the term "pharmaceutically acceptable salts" is intended to mean salts that retain the desired biological activity of the compounds and exhibit minimal or no undesired toxicological effects. Examples of such salts include, but are not limited to, salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, palmoic acid, alginic acid, polyglutamic acid, naphthalenesulfonic acid, naphthalenedisulfonic acid, methanesulfonic acid, p-toluenesulfonic acid and polygalacturonic acid. Other salts include pharmaceutically acceptable quaternary salts known by those skilled in the art, which specifically include the quaternary ammonium salt of the formula --NR+Z--, wherein R is hydrogen, alkyl, or benzyl, and Z is a counterion, including chloride, bromide, iodide, --O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

The following example is intended to further illustrate an embodiment of the invention and is not intended to limit its scope.

### Example

### Treatment of hypoxia induced by moderate exercise at elevated altitude

A 40 year old 90 kg male in good health is transported from low elevation (< 100 meters above sea level) to an altitude of 3,000 meters above sea level and kept at rest there for 3 hours. At that point, the subject is tested for respiration rate, heart rate and extremity O₂. The subject is then put on a stationary exercise bike and allowed to ride the bike at a cadence of 40 rpm for one hour. The subject is again tested for respiration rate, heart rate and extremity O₂. Then the subject is transported back to low elevation. The following day, the subject is again transported to the altitude of 3,000 meters above sea level and administered a dosage of 5-hydroxymethyl-2-furfural that has previously been determined to produce in the subject a P₅₀ of hemoglobin for oxygen of 18 mm Hg under standard conditions. The subject is kept at rest there for 3 hours, then tested for respiration rate, heart rate and extremity O₂. The subject is then put on a stationary exercise bike and allowed to ride the bike at a cadence of 40 rpm for one hour. The subject is again tested for respiration rate, heart rate and extremity O₂. The data from the two days are then compared. It is expected that the measured parameters will be closer to normal on day 2 than on day 1.

## Claims

1. Hydroxymethyl-2-furfural (5-HMF) for use in treating altitudinal hypoxia in a human subject, in a dosage sufficient to provide in the human subject a P₅₀ of hemoglobin from 15 mm Hg to 20 mm Hg under standard conditions of 37°C, pH 7.4, carbon dioxide tension of 40 mm Hg at sea level.

2. 5-HMF for use according to claim 1, wherein the dosage is from 300 mg to 10,000 mg 5-HMF.

3. 5-HMF for use according to claim 1, wherein the dosage is from 300 mg to 5,000 mg 5-HMF.

4. 5-HMF for use according to claim 1, wherein the dosage is from 300 mg to 3,000 mg 5-HMF.

5. 5-HMF for use according to claim 1, wherein the human is administered from 300 mg to 1,500 mg 5-HMF.

6. 5-HMF for use according to claim 1, wherein the dosage is from 1,500 mg to 10,000 mg 5-HMF.

7. 5-HMF for use according to claim 1, wherein the dosage is from 1,500 mg to 5,000 mg 5-HMF.

8. 5-HMF for use according to claim 1, wherein the dosage is from 1,500 mg to 3,000 mg 5-HMF.

9. 5-HMF for use according to claim 1, wherein the dosage is from 3,000 mg to 10,000 mg 5-HMF.

10. 5-HMF for use according to claim 1, wherein the dosage is from 3,000 mg to 5,000 mg 5-HMF.

11. 5-HMF for use according to claim 1 wherein 5-HMF is administered orally, by injection, or by inhalation.

12. 5-HMF for use according to claim 1 wherein 5-HMF is administered daily.

## Patentansprüche

1. Hydroxymethyl-2-furfural (5-HMF) zur Verwendung in der Behandlung von Höhenhypoxie in einem menschlichen Subjekt, in einer Dosierung, die ausreichend ist, um das menschliche Subjekt mit einem P₅₀-Wert von Hämoglobin von 15 mm Hg bis 20 mm Hg unter Standardbedingungen von 37°C, pH 7,4, Kohlendioxidspannung von 40 mm Hg bei Normalnull zu versorgen.

2. 5-HMF zur Verwendung gemäß Anspruch 1, wobei die Dosierung von 300 mg bis 10.000 mg 5-HMF reicht.

3. 5-HMF zur Verwendung gemäß Anspruch 1, wobei die Dosierung von 300 mg bis 5.000 mg 5-HMF reicht.

4. 5-HMF zur Verwendung gemäß Anspruch 1, wobei die Dosierung von 300 mg bis 3.000 mg 5-HMF reicht.

5. 5-HMF zur Verwendung gemäß Anspruch 1, wobei dem Menschen 300 mg bis 1.500 mg 5-HMF verabreicht werden.

6. 5-HMF zur Verwendung gemäß Anspruch 1, wobei die Dosierung von 1.500 mg bis 10.000 mg 5-HMF reicht.

7. 5-HMF zur Verwendung gemäß Anspruch 1, wobei die Dosierung von 1.500 mg bis 5.000 mg 5-HMF reicht.

8. 5-HMF zur Verwendung gemäß Anspruch 1, wobei die Dosierung von 1.500 mg bis 3.000 mg 5-HMF reicht.

9. 5-HMF zur Verwendung gemäß Anspruch 1, wobei die Dosierung von 3.000 mg bis 10.000 mg 5-HMF reicht.

10. 5-HMF zur Verwendung gemäß Anspruch 1, wobei die Dosierung von 3.000 mg bis 5.000 mg 5-HMF reicht.

11. 5-HMF zur Verwendung gemäß Anspruch 1, wobei 5-HMF oral, durch Injektion oder durch Inhalation verabreicht wird.

12. 5-HMF zur Verwendung gemäß Anspruch 1, wobei 5-HMF täglich verabreicht wird.

## Revendications

1. Hydroxyméthyl-2-furfural (5-HMF) pour utilisation dans le traitement de l'hypoxie altitudinale chez un sujet humain, dans un dosage suffisant pour fournir au sujet humain une P₅₀ de l'hémoglobine de 15 mm d'Hg à 20 mm d'Hg dans des conditions standard de 37 °C, pH 7,4, tension du dioxyde de carbone de 40 mm d'Hg au niveau de la mer.

2. 5-HMF pour utilisation selon la revendication 1, dans lequel le dosage est de 300 mg à 10 000 mg de 5-HMF.

3. 5-HMF pour utilisation selon la revendication 1, dans lequel le dosage est de 300 mg à 5 000 mg de 5-HMF.

4. 5-HMF pour utilisation selon la revendication 1, dans lequel le dosage est de 300 mg à 3 000 mg de 5-HMF.

5. 5-HMF pour utilisation selon la revendication 1, dans lequel il est administré à l'être humain de 300 mg à 1 500 mg de 5-HMF.

6. 5-HMF pour utilisation selon la revendication 1, dans lequel le dosage est de 1 500 mg à 10 000 mg de 5-HMF.

7. 5-HMF pour utilisation selon la revendication 1, dans lequel le dosage est de 1 500 mg à 5 000 mg de 5-HMF.

8. 5-HMF pour utilisation selon la revendication 1, dans lequel le dosage est de 1 500 mg à 3 000 mg de 5-HMF.

9. 5-HMF pour utilisation selon la revendication 1, dans lequel le dosage est de 3 000 mg à 10 000 mg de 5-HMF.

10. 5-HMF pour utilisation selon la revendication 1, dans lequel le dosage est de 3 000 mg à 5 000 mg de 5-HM F.

11. 5-HMF pour utilisation selon la revendication 1 dans lequel le 5-HMF est administré par voie orale, par injection ou par inhalation.

12. 5-HMF pour utilisation selon la revendication 1 dans lequel le 5-HMF est administré quotidiennement.
